# EUROPEAN PATENT APPLICATION

(11) **EP 4 603 187 A1**
(43) Date of publication of application: **20.08.2025**
(21) Application number: 24382170.9
(22) Date of filing: 19.02.2024
(51) Int. Cl.: B01L 3/00, G01N 33/483, C12M 3/06, C12M 1/12

(54) **MICROFLUIDIC DEVICES, SYSTEMS, AND METHODS FOR OBTAINING A SIGNAL**

(71) Applicant: Vitala Technologies, S.L., 08221 Terrassa (ES); Fundació Institut de Bioenginyeria de Catalunya (IBEC), 08028 Barcelona (ES)
(72) Inventor: MARCO RIUS, Irene, 08028 BARCELONA (ES); ORTEGA MACHUCA, Maria Alejandra, 08038 BARCELONA (ES); PORTELA OTAÑO, Alejandro, 08173 SANT CUGAT DEL VALLÈS (ES); HERRERO GÓMEZ, Alba, 08001 BARCELONA (ES)
(74) Representative: ZBM Patents - Zea, Barlocci & Markvardsen

(57) **Abstract**

In one aspect, a microfluidic device is provided. The microfluidic device comprises an inlet channel, a chamber, a porous element configured to receive a sample, and an outlet channel. The porous element is configured to be inserted in the chamber through the opening until the porous element reaches a locking position.

In a further aspect, a system comprising the microfluidic device, a solution comprising a label, and an apparatus is provided.

In a further aspect, a method for obtaining a signal of the detection complex is provided.

## Description

The present disclosure relates to microfluidic devices, systems and methods for obtaining a signal of a detection complex.

### BACKGROUND

In conventional microfluidic devices and cell culture methods, samples are seeded in the microfluidic device. The microfluidic device is treated so that the sample is attached to the bottom wall of the microfluidic device. As a result, the morphology and spatial configuration of the sample (e.g., a biological sample) is changed.

Furthermore, scaffolds or matrices with open pores can be arranged in the microfluidic device for accommodating the sample.

When the sample or the scaffold is not attached to the bottom wall of the microfluidic device, injection of a solution through an inlet channel of the microfluidic device may displace the sample or the scaffold within the microfluidic device. As a result, there may be no diffusion of the solution within the sample or the scaffold. When the solution is injected, there is an increase in the flow rate inside the microfluidic device. The increase in the flow rate leads to the sample or the scaffold being displaced towards an outlet channel of the microfluidic device. Therefore, the sample or the scaffold can clog the outlet channel of the microfluidic device.

Examples of the present disclosure seek to at least partially reduce one or more of the aforementioned problems.

### SUMMARY

In one aspect, a microfluidic device is provided. The microfluidic device comprises an inlet channel; a chamber; a porous element, and an outlet channel. The chamber is fluidically connected to the inlet channel. The chamber comprises an opening. The porous element is configured to receive a sample. Furthermore, the porous element is configured to be inserted in the chamber through the opening until the porous element reaches a locking position. The outlet channel is fluidically connected to the chamber.

The configuration of the microfluidic device and the porous element may allow processing of a sample in a more standardized and reproducible way by ensuring correct perfusion of the sample (i.e., the solution effectively diffuses through the sample). The configuration of the microfluidic device with the porous element may allow the porous element to remain at a fixed position by using, for example, the physical principle of friction, i.e., the force that opposes relative motion between systems in contact. This physical principle may be applied in the present disclosure to configure the size of the porous element.

Particularly, the size of the porous element may substantially match the size of the opening of the chamber. Thus, the porous element may be inserted in the chamber through the opening. The porous member may be configured to ensure that, when a solution is injected into the chamber, a frictional force resulting from the contact between the porous member and the chamber is greater than the force generated by the solution flowing through the sample and the porous element. When the porous element is arranged at the locking position and when the solution is inserted or injected into the chamber, the porous element remains at a fixed position (i.e., the locking position) because of the frictional force exerted between the chamber and the porous element. The frictional force opposes the flow of the solution that is being injected into the chamber.

The porous element may be configured to receive or accommodate the sample for contacting the sample with the solution to be inserted through the inlet channel. Depending on how the sample is arranged on the porous element, the porous element holds or supports the sample against the flow generated by injecting the solution into the chamber through the inlet channel. The sample may comprise an analyte of interest.

When the solution is injected into the chamber, the sample contacts or presses against the porous element. The porous element may maintain the sample at a desired position relative to a bottom wall of the chamber during injection of the solution (i.e., the porous element may avoid that the sample is displaced when the solution is injected in the chamber). The sample is therefore perfused with the solution that flows into the chamber because the porous element supports or holds the sample at a desired position. Furthermore, the porous element may apply a uniform pressure across a contact surface of the sample.

Because the porous element supports or retains the sample to be infused and the porous element remains at a fixed position, this allows correctly perfusing or diffusing a solution through the sample. The porous element, which remains at the locking position, prevents the sample from moving of its position when the sample is perfused with the solution. Therefore, the inlet channel or outlet channel of the microfluidic device may not be clogged by the sample or a portion of the sample.

In some examples, the porous element may receive a matrix with open pores (e.g., a scaffold) in which the sample is arranged. The matrix, which is a three-dimensional construct, may be configured to receive the sample or analyte. Therefore, the porous element may be either configured to receive the sample or the matrix containing or accommodating the sample.

In some of these examples, the matrix is a gel comprising sodium carboxymethyl cellulose. For example, the gel may comprise between 0.5 and 5 wt% of sodium carboxymethyl cellulose, and more specifically 1 wt% of sodium carboxymethyl cellulose.

In these examples, the three-dimensional construct formed from e.g., 1% sodium carboxymethyl cellulose gel may serve as a matrix for accommodating a sample (e.g., cells or any sample comprising an analyte) within the pores of the three-dimensional construct. Such 1% construct may provide a good stability and pore size distribution. Surprisingly, it has been found that 0.5 and 5 wt% of sodium carboxymethyl cellulose, and more specifically 1 wt% of sodium carboxymethyl cellulose has a low affinity for cell attachment and good physical-chemical properties for e.g., Nuclear Magnetic Resonance (NMR) applications, Positron Emission Tomography (PET) applications. Therefore, the matrix may provide conditions mimicking a human or animal body, allowing the cells' structure (i.e., the analyte) to live and allowing the cells' structure (i.e., the analyte) to form spheroids (a three-dimensional configuration) and interact with other cells.

In some examples, the matrix (i.e., a three-dimensional construct), may be selected from at least one of the following: a ceramic construct, a polymer construct, a metal and / or alloy construct, and a composite construct.

Therefore, the matrix and the porous element may give rise to a synergistic effect of providing conditions mimicking a human or animal body while ensuring the integrity of the sample during injection of the solution because the sample remains at a fixed position.

Referring to the porous element, in some examples, the porous element may comprise a porous layer and a frame fixedly attached to the porous layer. The porous layer may comprise pores which allow the solution to be exited from the chamber after perfusing the sample or diffusing through the sample. An example of a porous layer may be a mesh. Thus, the porous element may facilitate interrogation of the sample by maintaining its position and by allowing its perfusion.

In some examples, the porous layer may comprise a top face, a bottom face, and a matrix with open pores. The matrix may be arranged on at least one face of the porous layer. For example, the matrix may be arranged on both faces of the porous layer.

In some examples, the porous element may be slidably inserted into the chamber through the opening and the frame of the porous element may engage with a wall of the chamber until the locking position is reached.

In some examples, the frame of the porous element may comprise a connecting element. As a result, the porous element may be assembled with a second porous element via the connecting elements.

When the microfluidic device may comprise a second porous element, the connecting element of the porous element may be connected to the connecting element of the second porous element so that the porous element and the second porous element forms a cavity. The cavity may be configured to receive the sample or the matrix accommodating the sample.

In some of these examples, the connecting element of the porous element and/or the second porous element may comprise an opening and/or a protrusion. Consequently, the protrusion of the connecting element of the porous element may be inserted into the opening of the connecting element of the second porous element.

In some of these examples, the protrusion of the connecting element of the porous element may be inserted into the opening of the connecting element of the second porous element; and the protrusion of the connecting element of the second porous element may be inserted into the opening of the connecting element of the porous element.

In the examples, the formed cavity may receive the sample or the matrix accommodating the sample. Therefore, the sample or the matrix accommodating the sample may be located, at least partially, inside the cavity.

Because the sample or the matrix accommodating the sample is accommodated between the porous layer of the porous element and the porous layer of the second porous element, the solution may diffuse through the sample or through the matrix accommodating the sample. The solution may exit the sample and the porous elements to the outlet channel. Because the sample is located between the porous layer of the porous element and the porous layer of the second porous element, the sample may remain at a fixed position and may be prevented from clogging the outlet channel. Consequently, there may be no increase in pressure due to a clogged outlet channel.

Referring to the solution to be injected, in some examples, the solution may comprise a hyperpolarized isotope label or a non-hyperpolarized isotope label. When there is injection of the solution containing the hyperpolarized isotope label or the non-hyperpolarized isotope label, the sample and the label (i.e., either the hyperpolarized isotope label or the non-hyperpolarized label) may form a detection complex that can be detected.

In some examples, to establish a standardized and reproducible analysis technique, capable of processing and handling multiple samples of identical or different analytes contacted with the solution in a batch-like and in-line manner, the microfluidic device may be provided with multiple chambers, each chamber being in fluid connection with the inlet channel.

In a further aspect, a system is provided. The system comprises a microfluidic device according to the examples of the disclosure. The microfluidic device is configured to receive a sample, the sample comprising an analyte. The system further comprises a solution comprising a label, the solution to be introduced in a chamber of the microfluidic device through an inlet channel of the microfluidic device so that the label is contacted with the analyte to form a detection complex. The microfluidic device further comprises an apparatus to obtain a signal of the detection complex.

It may be noted that the detection complex may be located at the chamber and the apparatus to obtain a signal may be any suitable apparatus for obtaining the signal of the detection complex. Examples of apparatus for obtaining the signal of the detection complex may be a Nuclear Magnetic Resonance (NMR) apparatus or a Positron Emission Tomography (PET) apparatus.

This system allows a more standardized and reproducible analysis technique by effectively ensuring perfusion of the sample while obtaining a signal of the detection complex. Furthermore, depending on the configuration of the microfluidic device, the system may be capable of processing and handling multiple samples of identical or different analytes contacted with a solution in a batch-like and in-line manner.

In some examples, the system may comprise a processing unit for determining a state of the analyte based on the obtained signal or a sequence of the obtained signal of the detection complex. Therefore, a versatile platform is created, capable of acquisition of a signal or data pertaining to a state of a sample or analyte in a non-invasive manner by perfusing the sample with the solution and using, e.g., an NMR apparatus or a PET apparatus.

In some examples, the NMR apparatus comprises a housing defining a target area for accommodating at least the analyte contacted with the label, a magnetic unit and at least one magnetic gradient unit for applying - during use - one or more magnetic field gradients in the target area, a radiofrequency pulse generation unit for applying one or more sets of radiofrequency pulses towards the target area, and a radiofrequency receiving unit for acquiring signals.

In these examples, the NMR apparatus may be configured to accommodate in the target area the microfluidic device with the sample comprising the analyte to be contacted with the label. The sample may be accommodated in the chamber. As a result, reproducibility and repeatability of obtaining NMR signals of an analyte contacted with a label may be improved.

Similarly, the PET apparatus may be configured to accommodate in a target area the microfluidic device with the sample comprising the analyte contacted with the label. The sample may be accommodated in the chamber. Therefore, reproducibility and repeatability of obtaining PET signals of an analyte contacted with a label may be improved.

In a further aspect, a method of obtaining a signal of a detection complex is provided. The method comprises providing a sample comprising an analyte; providing a microfluidic device comprising: a chamber comprising an opening; a porous element configured to receive the sample, wherein the porous element is configured to be inserted in the chamber through the opening; and introducing a solution comprising a label in the chamber for contacting the label with the analyte to form a detection complex.

According to this aspect, acquisition of a signal or data in a non-invasive manner may be achieved.

In some examples, wherein the obtained signal of the detection complex is an obtained NMR signal of the detection complex, the method of obtaining the NMR signal of the detection complex may comprise applying one or more magnetic field gradients to the detection complex using a magnetic unit and a magnetic gradient unit of the NMR apparatus; applying one or more sets of radiofrequency pulses to the detection complex using a radiofrequency pulse generation unit of the NMR apparatus; and obtaining from a radiofrequency receiving unit of the NMR apparatus NMR signals or a sequence of NMR signals over time in response to the one or more sets of radiofrequency pulses.

In some examples, the method of obtaining a signal of the detection complex may further comprise determining a state of the analyte based on the obtained signal or a sequence of the obtained signal of the detection complex.

The term "label" may be used to refer hyperpolarized isotope labels or hyperpolarized labels.

The term "analyte" may be used to refer to healthy and diseased cells of human or animal organs and tissues, human or animal cells (or cell fragments), organoids, corporal fluids from humans or animals and whole animals as well as cells and fluids from vegetables. It may be understood that by animals, we may refer to animals such as but not limited to human beings, but also animal beings, such as rats, rabbits, pigs, and mice.

The term "hyperpolarized isotope label" may be used to refer to nuclei with enhanced nuclear spin polarization such as ¹H, ³He, ⁷Li, ¹³C, ¹⁵N, ¹⁹F, ³¹P, ⁸³Kr, and ¹²⁹Xe.

The term "non-hyperpolarized isotope label" may be used to refer to nuclei that have a spin quantum number above 0 (e.g., 1/2, 1, 3/2, 2, 5/2, 3, 7/2, 9/2). Throughout the disclosure, the non-hyperpolarized isotope label may refer to an isotope label which is not hyperpolarized.

The term "state" may be used to refer to a physiological condition, e.g., the health or unhealth (or disease) states as well as any transition state from health to unhealth (or disease) of the target / analyte; or the health or unhealth (or disease) states as well as any transition state from health to unhealth (or disease) of the whole human or animal being or of the human or animal being from whom those targets or analytes were taken.

The term "wt%" may be used to refer to a weight percentage of a first component (e.g., sodium carboxymethyl cellulose) relative to the total weight of a second component (e.g., the sample matrix).

### BRIEF DESCRIPTION OF THE DRAWINGS

Non-limiting examples of the present disclosure will be described in the following, with reference to the appended drawings, in which:
Figs 1, 2a, 2b, 2c schematically represent microfluidic devices according to some examples of the disclosure;
Fig. 3 schematically represents a matrix for accommodating a sample according to an example of the disclosure;
Figs. 4a and 4b schematically represent a porous element according to an example of the disclosure;
Figs. 5a and 5b schematically represents microfluidic devices according to some examples of the disclosure;
Fig. 6 schematically represents a system for obtaining a signal; and
Fig. 7 schematically shows a block diagram of a method for obtaining a signal of a detection complex.

### DETAILED DESCRIPTION OF EXAMPLES

In the figures, the same reference signs have been used to designate the same or analogous elements.

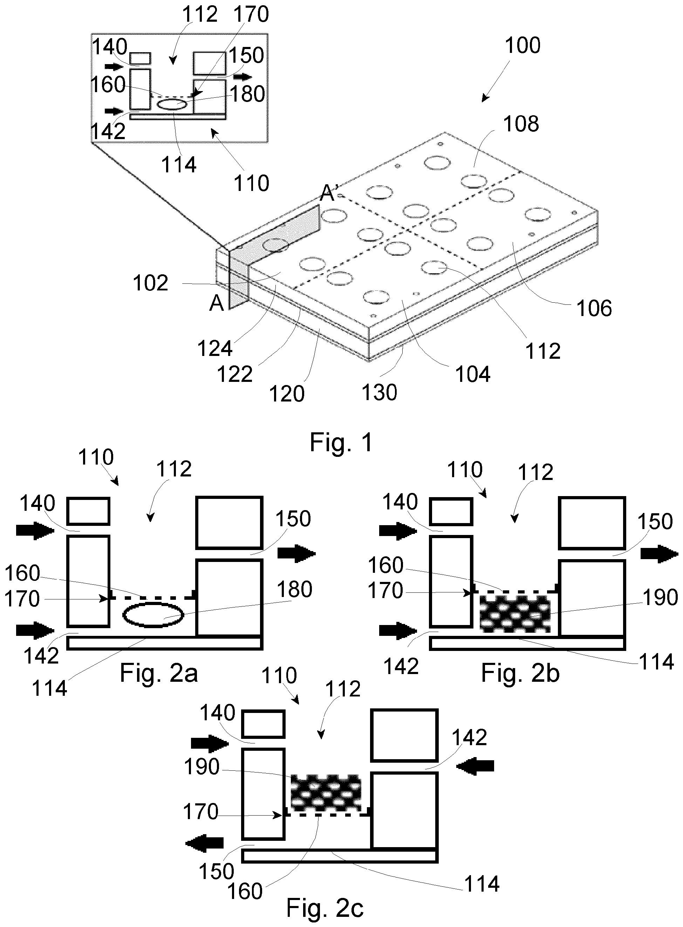

Fig. 1 shows a microfluidic device 100. The microfluidic device 100 comprises 4 regions 102, 104, 106 and 108, each region comprising one or more chamber 110. In fig. 1, four chambers 110 are shown in each region of the microfluidic device 100. Therefore, in the example of fig. 1, the microfluidic device is configured to receive up to 16 samples (1 sample per chamber). Consequently, the microfluidic device 100 may be capable of processing and handling multiple samples of identical or different analytes in a batch-like and in-line manner.

The microfluidic device 100 may be obtained by a stack of layers (e.g., a stack of 2 layers). The substrate may be composed of multiple polydimethylsiloxanes (PDMS) layers. In other examples, the microfluidic device may comprise glass and/or plastic.

For example, the PDMS layers may be manufactured by replica moulding using SU-8 microstructures produced by photolithography on 4-inch sized silicon substrates.

For manufacturing the PDMS layers forming the microfluidic device 100, the silicon substrates can be dehydrated using a hot plate at 200 °C for 30 minutes and afterwards cleaned and/or activated using an O₂ plasma (PDC-002, Harrick Plasma, Ithaca, NY, USA) treatment at 22.5 ml/min and 30 W for 20 minutes. A photoresist material (SU-8 2100, KAYAKU Advanced Materials, Inc., Westborough, MA, USA) can undergo spin-coating cycles to form a SU-8 layer of 200 µm thickness. The silicon substrate can be heated in-between and after the spin coating cycles in two steps: firstly at 65 °C for 5 min, and secondly at 95 °C for 20 min. The photoresistl can be exposed to UV-light at 320 mJ.cm⁻² (17.5 mW.cm⁻² for 18.2 s) using a mask aligner machine (MJB4, Süss MicroTec) and post-baked at 65 °C for 5 min followed by 95 °C for 14 min.

PDMS layers of the microfluidic device may be fabricated by soft lithography protocols using the patterned SU-8 moulds. PDMS prepolymer can be prepared in a ratio of 10:1 (base: curing agent, w/w) and degassed in a vacuum desiccator. The prepolymer can be cast on an element (e.g., a Petri dish) containing the SU-8 mould, heated at 65 °C for four hours, and subsequently left overnight at room temperature. As a result, PDMS layers 120, 122, 124 can be obtained. The PDMS layers 120, 122, 124 arranged on a glass side 130 can be activated using O₂ plasma and bonded together, resulting in a 11 mm thick microfluidic device 100.

Arranging the PDMS layers 120, 122, 124 on the glass side 130 is an example for manufacturing a microfluidic device 100. In some examples, manufacturing the microfluidic device 100 may not include a glass layer 130 on which the PDMS layers are arranged.

It may be noted that the microfluidic device 100 may comprise one chamber 110.

Fig. 1 shows a cross section along the plane AA' passing through the chamber 110 of the microfluidic device 100 (see inset at the left side of fig, 1). The microfluidic device 100 comprises an inlet channel 140, 142 and an outlet channel 150. The chamber 110 is fluidically connected to the inlet channel 140, 142. The chamber comprises an opening 112. The microfluidic device 100 further comprise a porous element 160. The porous element 160 may be inserted in the chamber 110 through the opening 112 until the porous element reaches a locking position 170. For example, the porous element 160 may be slidably inserted into the chamber 110. The outlet channel 150 is fluidically connected to the chamber 110.

The inset of fig. 1 shows a sample 180 located below the porous element 170 relative to a bottom wall 114 of the chamber 110. The sample 180 comprises an analyte of interest. Depending on the configuration of the microfluidic device 100 (i.e., location of the inlet channel 140, 142 and the outlet channel 150 relative to the sample), the porous element 160 may retain or support the sample 180 (as can be seen in figs. 2a, 2b and 2c).

Figs. 2a, 2b and 2c are cross sections along the plane AA' of fig. 1. Figs 2 a, 2b and 2c show an example of a configuration of the microfluidic device 100 where the porous element 160 is configured to receive the sample 180 (in fig. 2a) or a matrix 190 with open pores containing the sample 180 (in fig. 2b or fig. 2c).

In figs. 2a and 2b, when a solution is injected into the chamber 110 through the inlet channel 142, 144, the direction of the flow of the solution is from the bottom of the chamber 110 to the top of the chamber 110, towards the outlet channel 150 of the chamber. Consequently, for maintaining the sample 180 or the matrix 190 containing the sample, the porous element 160 is located above the sample 180 or the matrix 190 containing the sample. In figs. 2a and 2b, the locking position 170 is located above the inlet channel 140 with respect to the bottom wall 114 of the chamber 110; and the locking position 170 is located below the outlet channel 150 with respect to the bottom wall 114 of the chamber 110.

When the solution is injected into the chamber, the sample 180 or the matrix 190 containing the sample contacts or presses against the porous element 160. The porous element 160 may maintain the sample at a desired position relative to the bottom wall 114 of the chamber 110 during injection of the solution. Therefore, the sample 180 or the matrix 190 containing the sample is perfused with the solution that flows into the chamber 110 because the porous element 160 supports or holds the sample 180 or the matrix 190 containing the sample at a desired position. Furthermore, the porous element 160 may apply a uniform pressure across a contact surface of the sample (i.e., the surface of the sample or the surface of the matrix contacting the porous element).

In contrary, in fig. 2c, when a solution is injected into the chamber 110 through the inlet channel 140, 142, the direction of the flow of the solution is from the top of the chamber 110 to the bottom of the chamber 110, towards the outlet channel 150 of the chamber. In this example, the outlet channel 150 is located in the vicinity of the bottom wall 114. In fig. 2c, the locking position 170 is located below the inlet channel 140, 142 with respect to the bottom wall 114 of the chamber 110; and the locking position 170 is located above the outlet channel 150 with respect to the bottom wall 114 of the chamber 110. Consequently, for maintaining the sample 180 or the matrix 190 containing the sample, the porous element 160 is located below the sample 180 or the matrix 190 containing the sample. In the example of fig. 2c, the porous element 160 supports the matrix 190 containing the sample when the solution is injected.

Because the porous element 160 supports or retains the sample 180 or the matrix 190 containing the sample to be infused and the porous element remains at a fixed position, this allows correctly perfusing or diffusing a solution through the sample 180 or the matrix 190 containing the sample. The porous element 160, which remains at the locking position 170, allows to prevent the sample from moving of its position when the sample 180 or the matrix 190 containing the sample is perfused with the solution. Therefore, the inlet channel 140, 142 or the outlet channel 150 of the microfluidic device 100 may not be clogged by the sample or a portion of the sample.

The configuration of the microfluidic device with the porous element may allow the porous element to remain at a fixed position by using, for example, the physical principle of friction, i.e., the force that opposes relative motion between systems in contact. This physical principle may be applied in the present disclosure to configure the size of the porous element.

Particularly, the size of the porous element may substantially match the size of the opening of the chamber. Thus, the porous element may be inserted in the chamber through the opening. The porous member may be configured to ensure that, when a solution is injected into the chamber, a frictional force resulting from the contact between the porous member and the chamber is greater than the force generated by the solution flowing through the sample and the porous element.

When the porous element is arranged at the locking position and when the solution is inserted or injected into the chamber, the porous element remains at a fixed position (i.e., the locking position 170) because of a frictional force resulting from the contact between the porous member 160 and the chamber 110 is greater than the force generated by the solution flowing through e.g., the sample 180 and the porous element 160, or the matrix 190 containing the sample and the porous element 160.

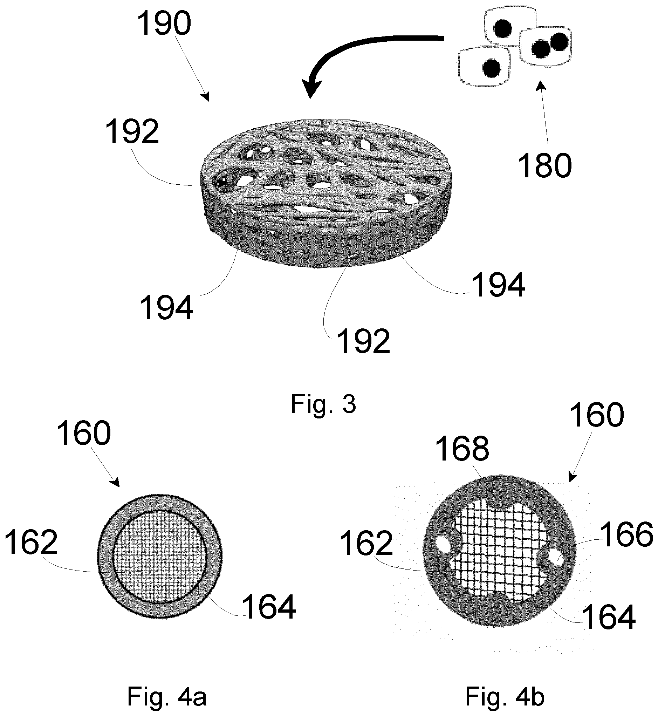

Fig. 3 shows the matrix 190 for accommodating the sample 180 which comprises an analyte of interest. In the example of fig. 3, the matrix 190 is formed as a three-dimensional construct. The three-dimensional construct of the matrix 190 defines a plurality of open pores 192 in which the sample 180 (e.g., a cluster of individual analytes or cells) may be accommodated.

The matrix 190 allows to study cell-cell interactions and tissue physiology without using laboratory animals or human subjects. Contrary to conventional cell culture, the sample 180 is not attached to e.g., the bottom wall 114 of the chamber 110 but accommodated in the open pores 192 of the matrix. Therefore, the morphology and function of the sample (e.g., cell) may not be changed.

In fig. 3, the three-dimensional construct of the matrix, 190 may be formed from a gel constituting a criss-cross lattice pattern of interconnecting gel strands 194 with open pores 192 in between. The open pores 192 may be of an irregular shape and size distribution.

In some examples, the three-dimensional construct of matrix 190 at least comprises sodium carboxymethyl cellulose and specifically at least comprises between 0.5 and 5% (wt.) sodium carboxymethyl cellulose, and more specifically 1% (wt.) sodium carboxymethyl cellulose.

The following method steps describe the steps required for the synthesis of e.g., 1% carboxymethyl cellulose gel.

Starting ingredients for fabrication of the 1% carboxymethyl cellulose gel may comprise:
- Sodium carboxymethyl-cellulose (CMC) [419273, Sigma]
- Adipic acid dihydrazide (AAD) [MW. = 174.2 g/mol]
- Morpholino-ethane-sulfonic acid (MES) [MW. = 195.2 g/mol], and
- N-(3-Dimethylaminopropyl)-N'-ethylcarbodiimide hydrochloride (EDC) [E7750-10g, Sigma]

In a first step, a MES buffer having a pH 5.5 and 0.5 M is prepared. For 100 ml, the steps are:
1.Prepare 80 ml of distilled H₂O in a container.
2.Add 9.76 g of MES free acid to the 80 ml of distilled H₂O solution.
3.Adjust the solution to a pH 5.5 using NaOH. For 100 ml, it may be required to add 366 mg of NaOH (solid) or 9.15 ml of NaOH 1 M to the distilled H₂O solution.
4.Add additional distilled H₂O until the volume of the solution has reached 100 ml.
In detail:

| MES | NaOH | distilled H₂O |
|---|---|---|
| 9-76 g | 366 mg | Adjust to 100 ml |

5.Subsequently, store the solution obtained in step 4 at 4 °C.
Next, a polymer premix is prepared (1.1 ml):
6.CMC 1% (w/v) is provided, and 50 mg thereof is dissolved in 5 ml of distilled H₂O, using stirring (e.g., with a magnetic stirrer at 900 rpm)
7.100 µL of AAD (50 mg/ml) is added to 1 ml of the CMC 1 % solution and mixed by means of pipetting.
8.This premix is pre-cooled at 4 °C.
9.4 mg of EDC is dissolved in 4 µl of distilled H₂O, thus preparing EDC (1 mg/µl).
10.The EDC is added to the premix, and the homogeneity of the solution is ensured by pipetting.
In detail:

| CMC 10 mg/mL (1 %) | MES pH 5.5, 0.5 M | d-H₂O | EDC (1 mg/mL) |
|---|---|---|---|
| 1 mL | 100 mL | 7 mL | 4 mL |

11.Next, the gel is transferred to the mould and incubated.
In examples of moulds with a large volume, the mould is pre-cooled in a freezer at -20 °C before the gel is added, thus:
12.Incubate at -20 °C. The mould containing the gel is to be placed in a freezer for 24 hours. After that time, the gel is to be removed carefully from the mould.

For example, a three-dimensional construct with open pores may be formed from 1% CMC gel, which construct may serve as the matrix 190 for accommodating the sample 180 comprising an analyte. Such 1% construct may provide a good stability and pore size distribution.

Surprisingly, it has been found that sodium carboxymethyl cellulose between 0.5 and 5 wt%, and more specifically 1 wt% of sodium carboxymethyl cellulose has a low affinity for cell attachment and good physical-chemical properties for, e.g., NMR applications or PET applications. Therefore, the three-dimensional construct of the matrix 190 may provide conditions mimicking a human or animal body, allowing the cells' structure (i.e., the sample comprising an analyte) to live and allowing the cells' structure (i.e., the sample comprising an analyte) to form spheroids (a three-dimensional configuration) and interact with each other.

An example of the matrix 190 obtained with the preparation technique described above may have a diameter of 5 mm and a height of 2 mm. More specifically, the dimensions of the matrix 190 may be in the range of 3 - 10 mm in diameter and in the range of 1 - 6 mm in height, depending on the intended application.

The matrix 190 increases the cell viability and optimizes the metabolic conditions required to study, for example, hepatocyte metabolism. The open, three-dimensional construct of the matrix 190 allows that sample 180 (such as cells) are retained inside the matrix 190 by the interconnecting gel strands 194 with open pores 192 between them.

In addition, it should be mentioned that the matrix 190 made from the CMC material as disclosed above, does not interfere during a signal acquisition (e.g., NMR signal or PET signal) and allows the correct perfusion of the solution. It may be noted that the matrix 190 may receive a sample 180 comprising an analyte of interest.

Figs. 4a and 4b schematically represent examples of porous element 160 to illustrate features of the present disclosure.

In the example of fig. 4a, the porous element 160 comprises a porous layer 162 (e.g., a mesh) and a frame 164 fixedly attached to the porous layer. The frame 164 of the porous element 160 engages with a wall of the chamber 110 until the locking position 170 is reached.

In some examples, the wall of the chamber 110 may comprise a groove for at least partially receiving a portion of the frame 164 of the porous element. In some of these examples, the groove may surround the wall of the chamber 110. Consequently, the frame 164 may remain at a fixed position (i.e., the locking position) when the frame reaches and enters the groove (that marks the locking position).

An example of manufacturing the porous element 160 of fig. 4a may be: 3D printing the frame 164 onto e.g., a nylon mesh with a pore size of 100µm (Manufac.). 3D printing may be carried by using a biocompatible 3D printing resin (SolusArt v3.0, Gesswein, USA) following a fabrication process using a Digital Light Processing 3D printer (DLP, Microlay, Versus 385nm, Spain). For example, a sheet of the nylon mesh may be cut to the size of the 3D printer build platform and fixed with special double-side adhesive tape (No.99786, 3M, USA) of 1cm width and 0.3 mm thickness on the four edges of the Building Platform. Subsequently, the 3D resin may be cured layer by layer following the CAD design of a ring of 6mm outer diameter and 5.5 mm internal diameter resulting in an embedded nylon mesh inside a rigid ring-shape frame formed by the cured 3D resin.

In some examples, the frame 164 of the porous element 160 may comprise a connecting element.

Fig. 4b shows an example of the frame 164 comprising such connecting elements. In the example of fig. 4b, the connecting element comprises an opening 166 and a protrusion 168.

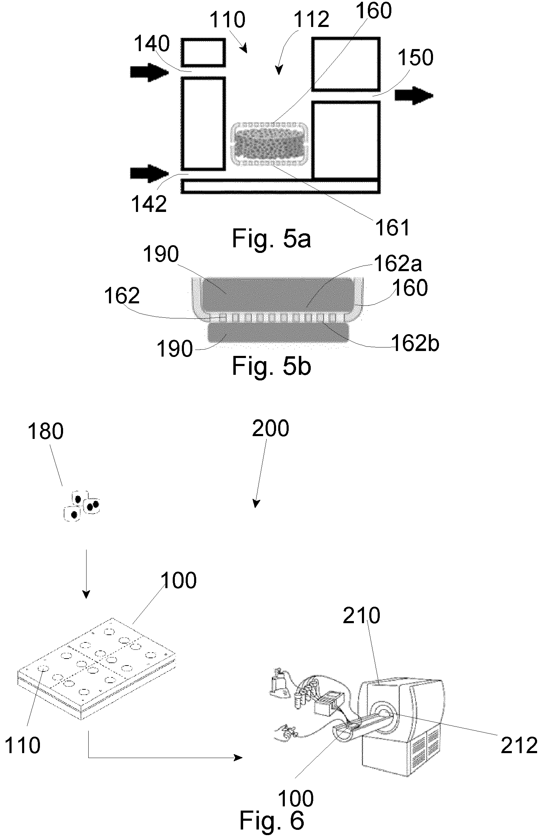

Fig. 5a schematically represent examples of the microfluidic device 100 to illustrate features of the present disclosure.

In the example of fig. 5a, the microfluidic device comprises a second porous element 161. The second porous element comprises the same feature than the porous element 160 as described above.

In fig. 5a, the connecting element of the porous element 160 is connected to the connecting element of the second porous element 161 so that the porous element and the second porous element forms a cavity. The cavity is formed between the porous layer of the porous element 160 and the porous layer of the second porous element 161.

In some examples, the connecting element of the porous element 160 may comprise a protrusion, and the connecting element of the second porous element 161 may comprise an opening. Therefore, in this example, the protrusion of the connecting element of the porous element 160 may be inserted into the opening of the connecting element of the second porous element 161 for forming the cavity.

In some examples, the sample 180 or the matrix 190 comprising the sample 180 may be located, at least partially, inside the cavity.

In some examples, the microfluidic device 100 may comprise a stack of an assembly comprising the porous element 160 and the second porous element 161. For example, there may be three assemblies inside the chamber 100.

Fig. 5b schematically represent examples of the microfluidic device 100 to illustrate features of the present disclosure.

In the example of fig. 5b, the porous layer 162 of the porous element 160 comprises a top face 162a and a bottom face 162b. In fig. 5b, the matrix 190 with open pores is arranged on at least one face of the porous layer 162. For example, a first portion of the matrix 190 with open pores may be arranged on the top face 162a of the porous layer 162, and a second portion of the matrix 190 with open pores may be arranged on the bottom face 162b of the porous layer 162.

Fig. 6 schematically represent examples of a system 200 for obtaining a signal to illustrate features of the present disclosure.

In fig. 6, the system 200 for obtaining a signal comprises the microfluidic device 100 according to an example of the present disclosure, a solution comprising a label, and an apparatus 210. The microfluidic device is configured to receive the sample 180 or the matrix 190 comprising the sample 180. The sample 180 or the matrix 190 are located in the chamber 110.

It may be noted that either the sample 180 or the matrix 190 comprising the sample 180 comprises an analyte of interest.

The solution is to be introduced in the chamber 110 of the microfluidic device 100 through the inlet channel 140, 142 so that the label is contacted with the analyte to form a detection complex. Consequently, the apparatus 210 that is configured to obtain a signal of the detection complex, obtains the signal of the detection complex which is located in the chamber 110.

In some examples, the apparatus 210 for obtaining the signal of the detection complex may be a Nuclear Magnetic Resonance (NMR) apparatus or a Positron Emission Tomography (PET) apparatus.

In some examples, the system 200 may further comprise a processing unit for determining a state of the analyte based on the obtained signal of the detection complex.

Referring to the solution to be injected, in some examples, the solution may comprise a hyperpolarized isotope label or a non-hyperpolarized isotope label. When there is injection of the solution containing the hyperpolarized isotope label or the non-hyperpolarized isotope label, the sample and the label (i.e., either the hyperpolarized isotope label or the non-hyperpolarized label) may form a detection complex that can be detected.

In some examples, depending on the apparatus to obtain the signal of the detection complex, the non-hyperpolarized isotope label may be a non-radioactive stable isotope. In some of these examples, the non-hyperpolarized isotope label may be Deuterium ²H. In addition, the non-hyperpolarized isotope label may be selected from at least one of the following: ²H, ⁶Li, ¹⁰B, ¹³C, ¹⁴N,¹⁵N, ¹⁹F, or ³¹P.

In some examples, the non-hyperpolarized isotope label may be radioactive depending on the apparatus to obtain the signal of the detection complex. An example of radioactive non-hyperpolarized isotope labels may be: ¹¹C, ¹³N, ¹⁵O, or ¹⁸F.

As a result, a radioactive non-hyperpolarized isotope label may contact the analyte to form the detection which may be a PET tracer used in positron emission tomography (PET).

Each PET tracer may comprise a positron-emitting isotope bound to an organic ligand. For example, a PET tracer may be fluorodeoxyglucose comprising a ¹⁸F radioactive non-hyperpolarized isotope label bound to e.g., 2-deoxy-2-glucose. The 2-deoxy-2-glucose ligand may be a substrate for an analyte of interest.

In some examples, wherein the apparatus to obtain the signal of the detection complex is a PET apparatus, the PET apparatus may comprise radiation detectors (i.e., scintillators), a photomultiplier, and read-out electronics.

The radioactive non-hyperpolarized isotope label may be a β+ radioactive atom comprising a short decay time, e.g., ¹¹C, ¹³N, ¹⁵O, or ¹⁸F. During decay of the nuclei of the radioactive non-hyperpolarized isotope labels, positrons may be emitted. When a positron is emitted and contacts an electron, the contact may produce gamma rays. The produced gamma rays may be detected by scintillators which may convert the gamma rays to photons of light that are transmitted to the photomultiplier which may convert the photons to electrical signals. These electrical signals may be processed by a processing unit to generate three dimensional images of the analyte. As a result, PET apparatuses may produce images of an analyte by detecting radiation emitted from the radioactive non-hyperpolarized isotope labels contacted with the analyte.

In some examples, the injection of the non-hyperpolarized isotope label may be performed via e.g., bolus injection (which may lead to high concentrations of non-hyperpolarized isotope label within seconds), or a continuous infusion of sample volume (which may lead to lower concentrations of non-hyperpolarized isotope label for over minutes to hours).

In fig. 6, the apparatus 210 (e.g., NMR apparatus or PET apparatus) is structured to accommodate in its target area 212, the microfluidic device 100 with the sample 180 or the matrix 190 comprising the sample 180 (both examples comprising an analyte of interest) infused with the solution injected in the chamber 110 through the inlet channel 140, 142. The system 200 is structured to determine a state of the analyte (e.g., healthy or unhealthy).

The apparatus 210, wherein the apparatus to obtain a signal of the detection complex is a NMR apparatus, also accommodates a magnetic unit and a magnetic gradient unit (not depicted) for applying - during use - one or more magnetic field gradients B₀ in the target area 212, to the detection complex. The NMR apparatus furthermore comprises in its housing a radiofrequency pulse generation unit for applying one or more sets of radiofrequency pulses towards the target area 212 to the detection complex comprising the one or multiple amounts of label with the analytes of interest.

In order to obtain spectroscopic information from the sample 180 or the matrix 190 comprising the sample 180 arranged on the chamber 110, radiofrequency pulse sequences are generated by a radiofrequency pulse generation unit and applied. These radiofrequency pulse sequences may provide spatially localized spectral information in a time-window of data acquisition.

The time-sequence set of NMR signals thus generated in the target area are picked up using a radiofrequency coil of the radiofrequency receiving unit of the NMR apparatus. The radiofrequency coil converts the change in the magnetic field in the target area 212 as induced by the nuclei's precession in the analytes into electromagnetic current signals and transferred to the processing unit. The processing unit (such as a processor) analyses the sequence of the obtained NMR signals and, based on the analysis, determines the state of the analyte.

Once the data-acquisition after the NMR measuring has been performed, the perfusion system starts again.

It may be noted that data-acquisition after the PET measuring may be performed in an analogue way to what is described above.

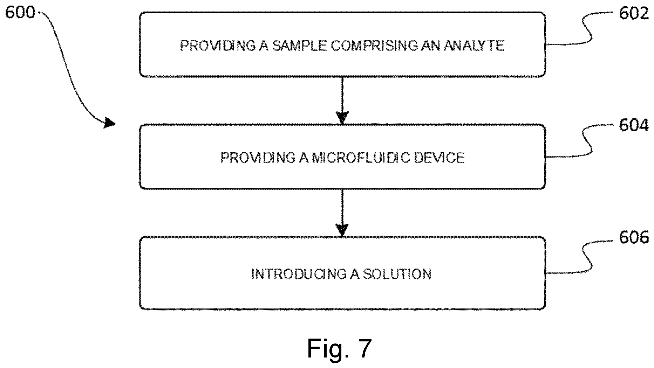

Fig. 7 schematically shows a block diagram of an example method 600 for obtaining a signal of the detection complex, to illustrate features of the present disclosure.

At block 602, the method 600 for obtaining a signal of a detection complex, the method comprises providing a sample comprising an analyte.

At block 604, the method 600 for obtaining the signal of the detection complex further comprises providing the microfluidic device 100 according to an example of the present disclosure.

As described above, the microfluidic device 100 comprises the chamber 110 comprising an opening 112; the porous element 160 configured to receive the sample 180 or the matrix 190 comprising the sample 180. Furthermore, the porous element 160 is configured to be inserted in the chamber 110 through the opening 112.

At block 606, the method 600 for obtaining the signal of the detection complex further comprises introducing a solution comprising a label in the chamber for contacting the label with the analyte to form a detection complex.

In some examples, obtaining the signal of the detection complex may comprise applying one or more magnetic field gradients to the detection complex using a magnetic unit and a magnetic gradient unit of an NMR apparatus; applying one or more sets of radiofrequency pulses to the detection complex using a radiofrequency pulse generation unit of the NMR apparatus; and obtaining from a radiofrequency receiving unit of the NMR apparatus a sequence of NMR signals over time in response to the one or more sets of radiofrequency pulses.

In some examples, the method 600 for obtaining the signal of the detection complex may further comprise determining a state of the analyte based on the obtained signal of the detection complex.

For reasons of completeness, various aspects of the present disclosure are set out in the following numbered clauses:
Clause 1. A microfluidic device comprising:
   an inlet channel;
   a chamber fluidically connected to the inlet channel, the chamber comprising an opening;
   a porous element configured to receive a sample, wherein the porous element is configured to be inserted in the chamber through the opening until the porous element reaches a locking position; and
   an outlet channel fluidically connected to the chamber.
Clause 2. The microfluidic device according to clause 1, wherein the porous element comprises:
   a porous layer;
   a frame fixedly attached to the porous layer.
Clause 3. The microfluidic device according to clause 2, wherein the frame of the porous element comprises a connecting element.
Clause 4. The microfluidic device according to clause 3, wherein the connecting element of the porous element comprises an opening and/or a protrusion.
Clause 5. The microfluidic device according to clause 3 or clause 4,
   wherein the microfluidic device comprises a second porous element,
   wherein the second porous element comprises a porous layer and a frame fixedly attached to the porous layer, the frame of the second porous layer comprising a connecting element; and
   wherein the connecting element of the porous element is connected to the connecting element of the second porous element so that the porous element and the second porous element forms a cavity.
Clause 6. The microfluidic device according to clause 5,
   wherein the connecting element of the porous element comprises a protrusion,
   wherein the connecting element of the second porous element comprises an opening; and
   wherein the protrusion of the connecting element of the porous element is inserted into the opening of the connecting element of the second porous element.
Clause 7. The microfluidic device according to clause 5 or clause 6, wherein the sample is located inside the cavity.
Clause 8. The microfluidic device according to any of clauses 2 to 7, wherein the frame of the porous element engages with a wall of the chamber until the locking position is reached.
Clause 9. The microfluidic device according to any of clauses 1 to 8,
   wherein the locking position is located above the inlet channel with respect to a bottom wall of the chamber; and
   wherein the locking position is located below the outlet channel with respect to the bottom wall of the chamber.
Clause 10. The microfluidic device according to any of clauses 1 to 8,
   wherein the locking position is located below the inlet channel with respect to a bottom wall of the chamber; and
   wherein the locking position is located above the outlet channel with respect to the bottom wall of the chamber.
Clause 11. The microfluidic device according to any of clauses 1 to 10,
   wherein the porous layer comprises:
   a top face and a bottom face; and
   a matrix with open pores, the matrix being arranged on at least one face of the porous layer, the matrix being configured for receiving the sample.
Clause 12. The microfluidic device according to clause 11, wherein the matrix is a gel comprising sodium carboxymethyl cellulose.
Clause 13. The microfluidic device according to clause 12, wherein the gel comprises between 0.5 and 5 wt% of sodium carboxymethyl cellulose.
Clause 14. The microfluidic device according to any of clauses 11 to 13, wherein the matrix is a three-dimensional construct; and wherein the three-dimensional construct is selected from at least one of the following: a ceramic construct, a polymer construct, a metal and / or alloy construct, or a composite construct.
Clause 15. A system comprising:
   a microfluidic device according to clauses 1 to 14 configured to receive a sample, the sample comprising an analyte;
   a solution comprising a label, the solution to be introduced in a chamber of the microfluidic device through an inlet channel of the microfluidic device so that the label is contacted with the analyte to form a detection complex; and
   an apparatus to obtain a signal of the detection complex.
Clause 16. The system according to clause 15, comprising:
   a processing unit for determining a state of the analyte based on the obtained signal of the detection complex.
Clause 17. The system according to clause 15 or clause 16,
   wherein the apparatus to obtain the signal of the detection complex is a Nuclear NMR or a PET apparatus.
Clause 18. A method for obtaining a signal of a detection complex, the method comprising:
   providing a sample comprising an analyte;
   providing a microfluidic device comprising:
      a chamber comprising an opening;
      a porous element configured to receive the sample, wherein the porous element is configured to be inserted in the chamber through the opening; and
   introducing a solution comprising a label in the chamber for contacting the label with the analyte to form a detection complex.
Clause 19. The method according to clause 18, wherein the obtaining the signal of the detection complex comprises:
   applying one or more magnetic field gradients to the detection complex using a magnetic unit and a magnetic gradient unit of an NMR apparatus;
   applying one or more sets of radiofrequency pulses to the detection complex using a radiofrequency pulse generation unit of the NMR apparatus; and
   obtaining from a radiofrequency receiving unit of the NMR apparatus a sequence of NMR signals over time in response to the one or more sets of radiofrequency pulses.
Clause 20. The method according to clause 18 or 19, the method comprising determining a state of the analyte based on the obtained signal of the detection complex.

## Claims

1. A microfluidic device comprising:
an inlet channel;
a chamber fluidically connected to the inlet channel, the chamber comprising an opening;
a porous element configured to receive a sample, wherein the porous element is configured to be inserted in the chamber through the opening until the porous element reaches a locking position; and
an outlet channel fluidically connected to the chamber.

2. The microfluidic device according to claim 1, wherein the porous element comprises:
a porous layer;
a frame fixedly attached to the porous layer.

3. The microfluidic device according to claim 2, wherein the frame of the porous element comprises a connecting element.

4. The microfluidic device according to claim 3, wherein the connecting element of the porous element comprises an opening and/or a protrusion.

5. The microfluidic device according to claims 3 or 4,
wherein the microfluidic device comprises a second porous element,
wherein the second porous element comprises a porous layer and a frame fixedly attached to the porous layer, the frame of the second porous layer comprising a connecting element; and
wherein the connecting element of the porous element is connected to the connecting element of the second porous element so that the porous element and the second porous element forms a cavity.

6. The microfluidic device according to claim 5,
wherein the connecting element of the porous element comprises a protrusion,
wherein the connecting element of the second porous element comprises an opening; and
wherein the protrusion of the connecting element of the porous element is inserted into the opening of the connecting element of the second porous element.

7. The microfluidic device according to claim 5 or claim 6, wherein the sample is located inside the cavity.

8. The microfluidic device according to any of claims 2 to 7, wherein the frame of the porous element engages with a wall of the chamber until the locking position is reached.

9. The microfluidic device according to any of claims 1 to 8,
wherein the locking position is located above the inlet channel with respect to a bottom wall of the chamber; and
wherein the locking position is located below the outlet channel with respect to the bottom wall of the chamber.

10. The microfluidic device according to any of claims 1 to 8,
wherein the locking position is located below the inlet channel with respect to a bottom wall of the chamber; and
wherein the locking position is located above the outlet channel with respect to the bottom wall of the chamber.

11. The microfluidic device according to any of claims 1 to 10,
wherein the porous layer comprises:
a top face and a bottom face; and
a matrix with open pores, the matrix being arranged on at least one face of the porous layer, the matrix being configured for receiving the sample.

12. The microfluidic device according to claim 11, wherein the matrix is a gel comprising sodium carboxymethyl cellulose.

13. A system comprising:
a microfluidic device according to claims 1 to 12 configured to receive a sample, the sample comprising an analyte;
a solution comprising a label, the solution to be introduced in a chamber of the microfluidic device through an inlet channel of the microfluidic device so that the label is contacted with the analyte to form a detection complex; and
an apparatus to obtain a signal of the detection complex.

14. The system according to claim 13, comprising:
a processing unit for determining a state of the analyte based on the obtained signal of the detection complex.

15. A method for obtaining a signal of a detection complex, the method comprising:
providing a sample comprising an analyte;
providing a microfluidic device comprising:
a chamber comprising an opening;
a porous element configured to receive the sample, wherein the porous element is configured to be inserted in the chamber through the opening; and
introducing a solution comprising a label in the chamber for contacting the label with the analyte to form a detection complex.
